# EUROPEAN PATENT APPLICATION

(11) **EP 2 638 904 A1**
(43) Date of publication of application: **18.09.2013**
(21) Application number: 12159348.7
(22) Date of filing: 13.03.2012
(51) Int. Cl.: A61K 31/352

(54) **Method for preventing or reducing postoperative adhesions**

(71) Applicant: Universiteit Maastricht, 6211 LK Maastricht (NL); Academisch Ziekenhuis Maastricht, 6229 HX Maastricht (NL)
(72) Inventor: Barneveld, van, Kevin Wijnand Yvo, 6221 JM MAASTRICHT (NL); Bouvy, Nicole Dorine, 6267 BW CADIER EN KEER (NL); Schreinemacher, Marc Henri Francois, 6229 ER MAASTRICHT (NL)
(74) Representative: Habets, Winand

(57) **Abstract**

This invention is in the field of experimental medicine. It provides means and methods for preventing or reducing postoperative adhesions. In particular, it provides a compound according to formula 1 for use in preventing or reducing postoperative adhesions wherein the compound is orally administered to a subject in need thereof immediately before surgery.

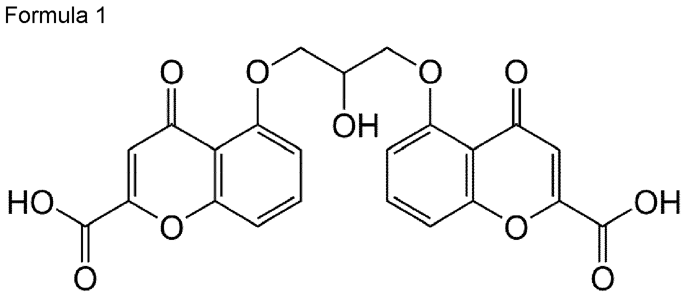

## Description

### Field of the invention

This invention is in the field of experimental medicine. It provides means and methods for preventing or reducing postoperative adhesions.

### Background of the invention

Postoperative adhesions occur in about 90% of all patients undergoing abdominal surgery and lead to at least one readmission for a third of these patients in the next 10 years [1, 2]. Adhesions become clinically apparent in the form of chronic abdominal pain, female infertility and small bowel obstruction [3, 4]. If reoperations are necessary, they are at a very high risk for inadvertent enterotomy leading to high morbidity and mortality [5, 6].

In thoracic surgery, especially the thoracotomy incision line is at risk for adhesions making it difficult to free the lung and to access the hilus at reoperation [7]. Likewise in cardiac surgery, adhesions may hinder reoperations at the level of the heart, great vessels or bypass conduits [8]. It is clear that here too, morbidity and mortality are raised.

Therefore, postoperative adhesions may not be regarded as a side effect of abdominal or thoracic surgery, but as the most common complication caused by surgery.

Incisional hernias occur in at least 10% of patients undergoing abdominal surgery [9]. Repair of these hernias includes mesh placement whenever possible in, order to reduce recurrence rates [10]. A position posterior to the abdominal musculature seems to be preferable and can be achieved in an extraperitoneal or intraperitoneal plane [11, 12]. In case of the latter, the mesh is an intraperitoneal foreign body and a specific leadpoint for adhesion formation.

It is highly desireable to prevent or reduce adhesions in general surgery, in particular in intraperitoneal mesh implantation [13].

Adhesion reduction is clearly of clinical interest. Besides reducing surgical trauma by meticulous surgical technique, powder free gloves, wetting tissues and operative time reduction, the use of adhesion barriers seems inevitable to obtain further adhesion prevention [14].

An adhesion barrier is a medical implant that can be used to reduce abnormal internal scarring (adhesions) following surgery, by separating the internal tissues and organs while they heal. Prior to the availability of adhesion barriers, adhesions were documented to be an almost unavoidable consequence of abdominal and pelvic surgery, and occurred in as much as 93% of all patients undergoing abdominal surgery [22]. These adhesions can lead to significant post-surgical morbidity including bowel obstruction, infertility, and chronic pelvic pain.

Surgeons and healthcare professionals developed several methods for minimizing tissue injury in order to minimize the formation of adhesions. However, even an experienced surgeon using advanced techniques may not be able to prevent the formation of adhesions following surgery without the aid of an adhesion barrier. Consequently, many surgeons have come to rely upon adhesion barriers for adhesion prevention following abdominal and pelvic surgery.

Adhesion barriers, such as Seprafilm, are films that are applied between layers of tissues at the end of a surgery before the incision site is closed. Seprafilm is a clear, sticky film composed of chemically modified sugars, some of which occur naturally in the human body. It sticks to the tissues to which it is applied and is slowly absorbed into the body over a period of seven days. While in place, Seprafilm acts as a physical barrier that separates traumatized tissue surfaces so that they do not adhere to one another while the tissue surfaces heal. Other adhesion barriers, such as Interceed, work in much the same way, with varying degrees of efficacy over varying lengths of time. Other technologies can be to separate the organs with gels, like Intercoat or Hyalobarrier.

A local barrier composed of hyaluronic acid and carboxymethylcellulose (Seprafilm®) has proven to be effective in reducing adhesions in various open general surgery studies [15]. Previous publications have also shown significant benefit of Interceed® membrane, composed of oxidized regenerated cellulose, in open gynecological surgery [16]. Administration of an icodextrin solution (Adept®) that spreads throughout the peritoneal cavity has shown adhesion reductive capacity in benign laparoscopic gynecological surgery [17]. In cardiothoracic surgery, mostly experimental studies using the same barriers as in abdominal surgery are tested. In intraperitoneal mesh implantation, layered coatings have shown a reduction in adhesion formation [18].

It is generally considered that some people are more prone to develop postoperative adhesions than are others. Unfortunately, there is no available marker to predict the occurrence or the extent and severity of adhesions preoperatively. Additionally, there are no available serum markers or imaging studies that are generally considered to be able to predict the incidence, severity, or extent of adhesions.

In conclusion, products that prevent or reduce postoperative adhesions comprise a local measure that has to be applied after the trauma has taken place. There is a great need for preventive measures against adhesions capable of attenuating or completely prohibiting the processes that are set into action by trauma. Unfortunately, such preventive measures are not available at present. There is a widely recognized need in the art for measures that prevent or reduce postoperative adhesions.

### Detailed description of the invention.

The invention provides means and methods for preventing or reducing postoperative adhesions by oral administration of a compound according to formula 1 to a subject in need of such a treatment before surgery.

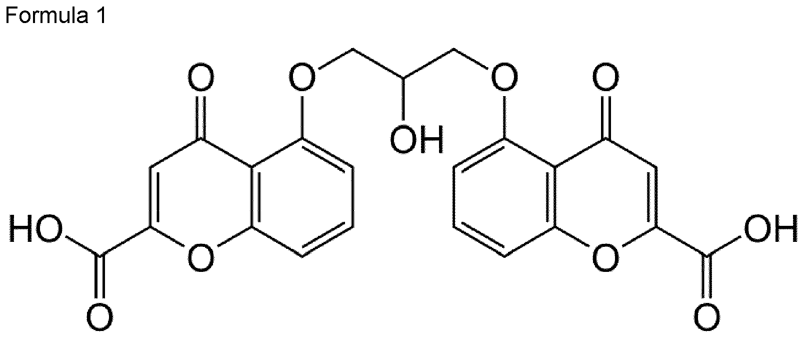

A compound according to formula 1 is known and commercially available under the brand name Cromolyn ®.

The invention may also be described as providing a compound according to formula 1 for use in preventing or reducing postoperative adhesions wherein the compound is orally administered to a subject in need thereof before surgery. In a preferred embodiment, the treatment is performed immediately before surgery, such as for instance less than 4 days, such as 2 days or 1 day before surgery, such as 20, 18, 16, 14, 12, 10, 8, 6, 4, 3, 2, 1 hour before surgery or even less.

As used herein, the term "adhesions" refers to are non anatomic connections of mostly fibrous tissue between normal peritoneal surfaces. Postoperative adhesions may be classified as de novo or re-formed (Table 1, Diamond MP, Nezhat F. Adhesions after resection of ovarian endometriomas. Fertil Steril 1993; 59: 934-935).

**Table 1. Classification of Postoperative Adhesion Development**

| | |
|---|---|
| **Type 1** | De novo adhesion formation; development of adhesions at sites that did not have adhesion initially |
| | A No operative procedure at site of adhesion formation |
| | B Operative procedure performed at site of adhesion formation |
| | Adhesion re-formation; redevelopment of adhesions at sites at which adhesiolysis was performed |
| **Type 2** | A No operative procedure at site of adhesion re-formation (other than adhesiolysis) |
| | B Operative procedure performed at site of adhesion re-formation (in addition to adhesiolysis) |

De novo, or type 1, adhesions are adhesions that develop (i.e., are newly formed) at sites that did not have adhesions at an initial surgical procedure. In contrast, re-formed, or type 2, adhesions are classified as adhesions that develop at the sites of previous adhesiolysis.

Types 1 and 2 may further be classified into A and B subgroups. Subgroup A adhesions are seen at the sites where no operative procedure was performed (type 1A) excluding prior adhesiolysis (type 2A). In contrast, subgroup B adhesions develop at the sites that underwent surgical procedures without adhesiolysis (type 1B; e.g., myomectomy, ovarian cystectomy, etc.) or in addition to adhesiolysis (type 2B).

The term adhesiolysis in this context is to be interpreted as the surgical lysis of adhesions, for example by laparoscopy.

Adhesions form as a natural part of the body's healing process after surgery in the same way that a scar forms. The term "adhesion" is applied when the scar extends from within one tissue across to another, usually across a virtual space, such as for example the peritoneal cavity. As part of the process, the body deposits fibrin onto injured tissues. The fibrin acts like a glue to seal the injury and builds the fledgling adhesion, said at this point to be "fibrinous". In body cavities such as the peritoneal, pericardial and synovial cavities, a family of fibrinolytic enzymes may act to limit the extent of the initial fibrinous adhesion, and may even dissolve it. In many cases however the production or activity of these enzymes are compromised because of injury, and the fibrinous adhesion persists. If this is allowed to happen, tissue repair cells such as macrophages, fibroblasts and blood vessel cells, penetrate into the fibrinous adhesion, and lay down collagen and other matrix substances to form a permanent fibrous adhesion.

While some adhesions do not cause problems, others can prevent muscle and other tissues and organs from moving freely, sometimes causing organs to become twisted or pulled from their normal positions.

In summary, adhesions are mostly fibrous bands that may form between tissues and organs, often as a result of injury during surgery. They may be thought of as internal scar tissue that connect tissues not normally connected.

Abdominal adhesions (or intra-abdominal adhesions) are most commonly caused by abdominal surgical procedures but may also be caused by pelvic inflammatory disease or endometriosis. The adhesions start to form immediately after surgery, such as for example within hours after surgery and may cause internal organs to attach to the surgical site or to other organs in the abdominal cavity. Adhesion-related twisting and pulling of internal organs can result in complications such as infertility and chronic pelvic pain.

Surgery inside the uterine cavity (e.g., suction D&C, myomectomy, endometrial ablation) may result in Asherman's Syndrome (also known as intrauterine adhesions), a cause of infertility.

Small bowel obstruction (SBO) is another significant consequence of post-surgical adhesions. A SBO may be caused when an adhesion pulls or kinks the small intestine and prevents the flow of content through the digestive tract. It can occur 20 years or more after the initial surgical procedure, if a previously benign adhesion allows the small bowel to spontaneously twist around itself and obstruct. SBO is an emergent, possibly fatal condition without immediate medical attention. According to statistics provided by the National Hospital Discharge Survey approximately 2,000 people die every year in the USA from obstruction due to adhesions. Depending on the severity of the obstruction, a partial obstruction may relieve itself with conservative medical intervention. However, many obstructive events require surgery to lyse the offending adhesion(s) or resect the affected small intestine.

A recent study showed that more than 90% of patients develop adhesions following open abdominal surgery and 55%-100% of women develop adhesions following pelvic surgery.[19] Adhesions from prior abdominal or pelvic surgery can obscure visibility and access at subsequent abdominal or pelvic surgery. In a study of 29,790 participants, 35% of patients who underwent open abdominal or pelvic surgery were readmitted to the hospital for an average of two times after their surgery, due to adhesion-related or adhesion-suspected complications.[20] Over 22% of all re-admissions occurred in the first year after the initial surgery.[20] Adhesion-related complexity at reoperation adds significant risk to subsequent surgical procedures.[21].

There are two types of adhesions that may be distinguished. First there are fibrinous adhesions which are causative of early postoperative obstruction which settles down within 3-5 days. The majority of fibrinous adhesions will disappear in due course of time.

Second there are Fibrous adhesions. If the infection is continuous or if foreign material is present, the fibrinous material is converted into fibrous material.

A manual manipulative physical therapy (The Wurn Technique) applied to the body's soft tissues has been examined as a nonsurgical treatment to decrease adhesions causing pain, infertility, or dysfunction. In a study on the rate of natural pregnancy within one year for infertile women who received the Wurn Technique (average infertility five years), 71% [10/14] became pregnant.[23] In a second study in 2004, the therapy improved pregnancy rates for women undergoing in vitro fertilization (IVF) procedures. Women who received the therapy within 15 months before an IVF transfer had a 67% pregnancy rate vs. the 41% US Center for Disease Control national average for IVF.[23] All study participants had histories indicating abdominopelvic adhesion formation.[23]

We have undertaken experiments that show that pre-operative administration of Cromolyn ® reduces adhesions after intraperitoneal mesh placement. In all experiments Balb/c mice (n=6-9/group) received an uncoated polypropylene mesh intraperitoneally. Adhesions were evaluated by the percentage of the actual mesh surface covered with adhesions. Images of the meshes were computer analyzed by preparing a digital overlay of the images outlining adhesions and the total mesh surface. All data are expressed as means (standard error of means).

Cromolyn dissolved in 0.9% NaCl was administered orally by oral gavage per 0.4ml bolus or (as a control) intraperitoneally by injection. Sodium chloride 0.9% served as control. Administration was commenced 2 days before the operation and continued until 1 or 7 days postoperatively. Animals were sacrificed at 7 days follow-up.

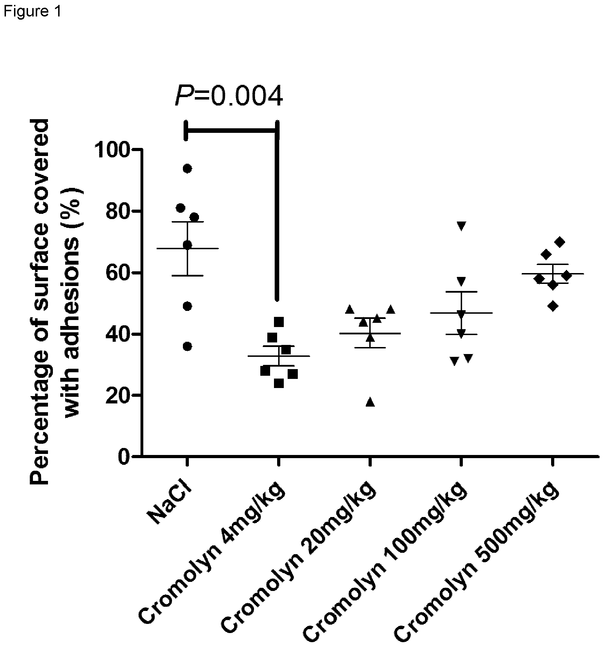

We found that oral administration of Cromolyn remarkably reduced the adhesion after surgery, as expressed in a percentage of mesh surface covered with adhesion. Figure 1 shows that an oral dose of 4mg/kg significantly reduced the mean surface from 68% +/- 9% to 33% +/- 3%. Interestingly, it appeared that a lower dose of Cromolyn showed better results than higher doses. The optimum dose may effectively be determined by a skilled person and may deviate somewhat from species to species. In general however, a dose of 20 mg/kg bodyweight or below is preferred, such as 10, 8, 4, 2, 1, 0,5, 0,1 mg/kg bodyweight or below is preferred. Misschien hoeft het maar 1 keer preoperatief gegeven te worden. Dat experiment wordt nu gedaan. Dient dat nog explicieter vermeld te worden?

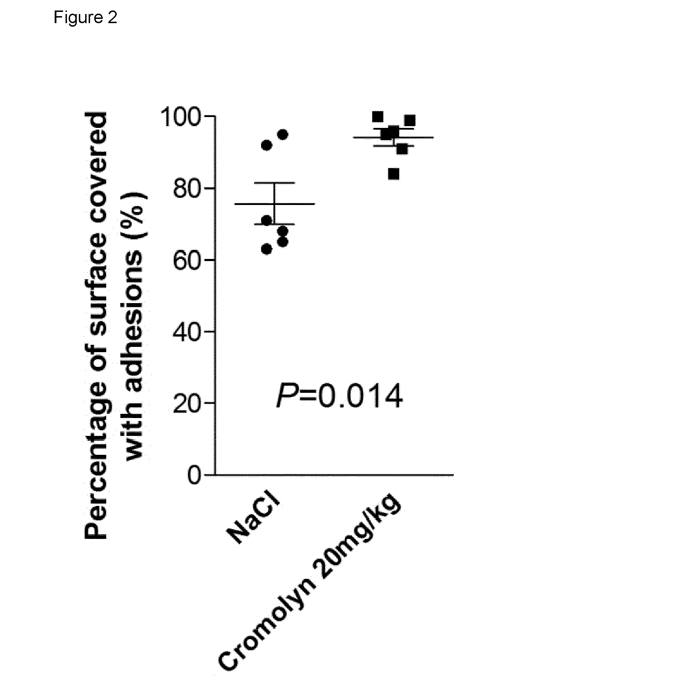

When administered intraperitoneally until 7 days after surgery, Cromolyn ® appeared to have the opposite effect; it increased the surface area covered with adhesions from 76% ± 6 to 94% ± 2 (figure 2).

Cromolyn ® is also referred to as Cromoglicic acid, cromoglycate, or cromoglicate and is traditionally described as a mast cell stabilizer, and is commonly marketed as the sodium salt sodium cromoglicate or cromolyn sodium. This drug prevents the release of inflammatory chemicals such as histamine from mast cells.

Because of their convenience (and perceived safety), leukotriene receptor antagonists have largely replaced it as the non-corticosteroid treatment of choice in the treatment of asthma. Cromoglicic acid requires administration four times daily, and does not provide additive benefit in combination with inhaled corticosteroids..

The systematic IUPAC name of Cromolyn is 5,5'-(2-hydroxypropane-1,3-diyl)bis(oxy)bis(4-oxo-4H-chromene-2-carboxylic acid)), a structure according to formula 1 above.

It has been used in a study towards the effects of mast cell modulation on early host response to implanted synthetic meshes [24]. In this experimental study, meshes were implanted subcutaneously (extraperitoneally). Cromolyn was administered intraperiteonally from 3 days preoperatively until two weeks postoperatively at which point mice were sacrificed. The fibrotic response to the meshes was diminished. This study does not describe the oral administration of Cromolyn ®.

In an experimental study towards the role of mast cells in peritoneal adhesion formation [25], adhesions were induced by traumatizing the cecum and instilling 95% ethanol intraperitoneally. Intraperitoneal administration of Cromolyn ® 30 min before operation and once postoperatively reduced intraperitoneal adhesions significantly at one week follow-up. This study does not disclose the oral administration of Cromolyn ®.

In another study [26], a trial was performed aimed at reducing adhesion formation in a uterine horn model. In this experimental study, adhesions were induced on rats's uterine horn. Intraperitoneal administration of cromolyn 30 min before operation and at the end of the operation did not reduce adhesions at two weeks follow-up. This study does not disclose the oral administration of Cromolyn ®.

In another study (27), adhesions were induced by traumatizing the cecum. Intraperitoneal administration of cromolyn 30 min before operation and right after the operation reduced adhesions. This study does not disclose the oral administration of Cromolyn ®.

In a further study directed towards the prevention of adhesions by sodium cromoglycate, dexamethasone, saline and aprotinin after pelvic surgery, adhesions were induced on rats's uterine horn. Intraperitoneal administration of Cromolyn ® at the end of the operation reduced adhesions at ten days follow-up. This study does not disclose the oral administration of Cromolyn ®.

We concluded from our experiments that Cromolyn ® effectively prevents or reduces postoperative adhesions when administered orally.

The invention therefore relates to a method for preventing or reducing postoperative adhesions by oral administration of a compound according to formula 1 to a subject in need of such a treatment before surgery.

The invention may also be described as a compound according to formula 1 for use in preventing or reducing postoperative adhesions wherein the compound is orally administered to a subject in need thereof immediately before surgery.

The invention may advantageously be practiced in those cases wherein the postoperative adhesions are adhesions between tissues or adhesions between a tissue and a foreign body. Foreign bodies may be a variety of implants, meshes, or the like, advantageously, the foreign body is a mesh.

Best results were achieved with adhesions in the thorax or abdomen. The invention is preferably performed in thoracal or abdominal surgery.

The oral dose may be administered in any way suitable. It is preferred to continue the adminstration of the compound during surgery and/or for a certain amount of time after surgery. The invention therefore relates to a compound for use as described above, wherein the administration is continued until after the surgery is completed or even is continued at least until 1 day after the surgery, such as for instance until 7 days after surgery.

### Legend to the figures

### Figure 1 Oral administration of Cromolyn.

Oral administration of several doses of Cromolyn reduced adhesion. The figure shows that an oral dose of 4mg/kg significantly reduced the mean surface of adhesions to a mesh from 68% +/- 9% to 33% +/- 3%.

### Figure 2 Intraperitoneal administration of Cromolyn ®.

When administered intraperitoneally until 7 days after surgery, Cromolyn ® appeared to induce adhesion, as evidenced by an increased surface area covered with adhesions (from 76% ± 6 to 94% ± 2) All data are expressed as means (standard error of means).

### Literature incorporated by reference

1. Ellis H, Moran BJ, Thompson JN, Parker MC, Wilson MS, Menzies D, McGuire A, Lower AM, Hawthorn RJ, O'Brien F, Buchan S, Crowe AM. Adhesion-related hospital readmissions after abdominal and pelvic surgery: a retrospective cohort study. Lancet 1999; 353(9163): 1476-1480.
2. Parker MC, Ellis H, Moran BJ, Thompson JN, Wilson MS, Menzies D, McGuire A, Lower AM, Hawthorn RJ, O'Briena F, Buchan S, Crowe AM. Postoperative adhesions: ten-year follow-up of 12,584 patients undergoing lower abdominal surgery. Dis Colon Rectum 2001; 44(6): 822-829; discussion 829-830.
3. Sulaiman H, Gabella G, Davis MC, Mutsaers SE, Boulos P, Laurent GJ, Herrick SE. Presence and distribution of sensory nerve fibers in human peritoneal adhesions. Ann Surg 2001; 234(2): 256-261.
4. Vrijland WW, Jeekel J, van Geldorp HJ, Swank DJ, Bonjer HJ. Abdominal adhesions: intestinal obstruction, pain, and infertility. Surg Endosc 2003; 17(7): 1017-1022.
5. Van Der Krabben AA, Dijkstra FR, Nieuwenhuijzen M, Reijnen MM, Schaapveld M, Van Goor H. Morbidity and mortality of inadvertent enterotomy during adhesiotomy. Br J Surg 2000; 87(4): 467-471.
6. Wullstein C, Gross E. Laparoscopic compared with conventional treatment of acute adhesive small bowel obstruction. Br J Surg 2003; 90(9): 1147-1151.
7. Karacam V, Onen A, Sanli A, Gurel D, Kargi A, Karapolat S, Ozdemir N. Prevention of pleural adhesions using a membrane containing polyethylene glycol in rats. Int J Med Sci 2011; 8(5): 380-386.
8. Bel A, Ricci M, Piquet J, Bruneval P, Perier MC, Gagnieu C, Fabiani JN, Menasche P. Prevention of postcardiopulmonary bypass pericardial adhesions by a new resorbable collagen membrane. Interact Cardiovasc Thorac Surg 2012.
9. Kuhry E, Schwenk WF, Gaupset R, Romild U, Bonjer HJ. Long-term results of laparoscopic colorectal cancer resection. Reviews 2008(2): CD003432.
10. Burger JW, Luijendijk RW, Hop WC, Halm JA, Verdaasdonk EG, Jeekel J. Long-term follow-up of a randomized controlled trial of suture versus mesh repair of incisional hernia. Ann Surg 2004; 240(4): 578-583; discussion 583-575.
11. Israelsson LA, Smedberg S, Montgomery A, Nordin P, Spangen L. Incisional hernia repair in Sweden 2002. Hernia 2006; 10(3): 258-261.
12. Hawn MT, Snyder CW, Graham LA, Gray SH, Finan KR, Vick CC. Long-term follow-up of technical outcomes for incisional hernia repair. J Am Coll Surg 2010; 210(5): 648-655, 655-647.
13. Schreinemacher MH, Ten Broek RP, Bakkum EA, van Goor H, Bouvy ND. Adhesion Awareness: A National Survey of Surgeons. World J Surg 2010.
14. van den Tol MP, Haverlag R, van Rossen ME, Bonthuis F, Marquet RL, Jeekel J. Glove powder promotes adhesion formation and facilitates tumour cell adhesion and growth. Br J Surg 2001; 88(9): 1258-1263.
15. Kumar S, Wong PF, Leaper DJ. Intra-peritoneal prophylactic agents for preventing adhesions and adhesive intestinal obstruction after non-gynaecological abdominal surgery. Reviews 2009(1): CD005080.
16. Wiseman DM, Trout JR, Franklin RR, Diamond MP. Metaanalysis of the safety and efficacy of an adhesion barrier (Interceed TC7) in laparotomy. The Journal of reproductive medicine 1999; 44(4): 325-331.
17. Brown CB, Luciano AA, Martin D, Peers E, Scrimgeour A, diZerega GS. Adept (icodextrin 4% solution) reduces adhesions after laparoscopic surgery for adhesiolysis: a double-blind, randomized, controlled study. Fertil Steril 2007; 88(5): 1413-1426.
18. Schreinemacher MH, Emans PJ, Gijbels MJ, Greve JW, Beets GL, Bouvy ND. Degradation of mesh coatings and intraperitoneal adhesion formation in an experimental model. Br J Surg 2009; 96(3): 305-313.
19. Liakakos, T; Thomakos, N; Fine, PM; Dervenis, C; Young, RL (2001). "Peritoneal adhesions: etiology, pathophysiology, and clinical significance. Recent advances in prevention and management". Digestive surgery 18 (4): 260-73
20. Ellis, H; Moran, BJ; Thompson, JN; Parker, MC; Wilson, MS; Menzies, D; McGuire, A; Lower, AM et al (1999). "Adhesion-related hospital readmissions after abdominal and pelvic surgery: a retrospective cohort study". Lancet 353 (9163): 1476-80.
21. Van Der Krabben, AA; Dijkstra, FR; Nieuwenhuijzen, M; Reijnen, MM; Schaapveld, M; Van Goor, H (2000). "Morbidity and mortality of inadvertent enterotomy during adhesiotomy". The British journal of surgery 87 (4): 467-71.
22. Adhesion prevention: a standard of care. 1999 - 2003 Medical Association Communications. American Society of Reproductive Medicine. http://www.cmecorner.com/macmcm/asrm/asrm2002_02.htm
23. Wurn, BF; Wurn, LJ; King, CR; Heuer, MA; Roscow, AS; Scharf, ES; Shuster, JJ (2004). "Treating Female Infertility and Improving IVF Pregnancy Rates With a Manual Physical Therapy Technique". MedGenMed : Medscape general medicine 6 (2): 51..
24. Orenstein SB, Saberski ER, Klueh U, Kreutzer DL, Novitsky YW. Hernia. 2010 Oct;14(5):511-6. Epub 2010 Jun 5.
25. Liebman SM, Langer JC, Marshall JS, Collins SM. Am J Surg. 1993 Jan;165(1):127-30.
26. Vural B, Mercan R, Corakçi A, Ozeren S, Keskin N, Vural S, Yücesoy I, Erk A. Gynecol Obstet Invest. 1998;45(1):58-61.
27. Cantürk NZ, Vural B, Cubukcu A, Duzcen E, Utkan Z, Dülger M. East Afr Med J. 1999 Apr;76(4):233-6.
28. Kucukozkan T, Ersoy B, Uygur D, Gundogdu C. ANZ J Surg. 2004 Dec;74(12):1111-5.

## Claims

1. A compound according to formula 1 for use in preventing or reducing postoperative adhesions wherein the compound is orally administered to a subject in need thereof before surgery.

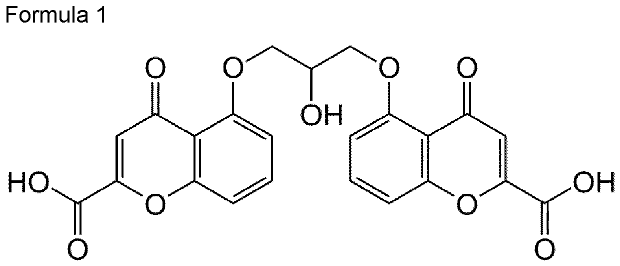

2. A compound for use according to claim 1 wherein the postoperative adhesions are adhesions between tissues or adhesions between a tissue and a foreign body.

3. A compound for use according to claims 1 or 2 wherein the adhesions are adhesions in the thorax or abdomen.

4. A compound for use according to any of claims 1-3 wherein the surgery is thoracal or abdominal surgery.

5. A compound for use according to any of claims 2-4 wherein the foreign body is a mesh.

6. A compound for use according to any of claims 1-5 wherein the administration is continued until after the surgery is completed.

7. A compound for use according to claim 6 wherein the administration is continued at least until 1 day after the surgery.

8. A compound for use according to claim 7 wherein the administration is continued at least until 7 days after surgery.
